# EUROPÄISCHE PATENTSCHRIFT

(11) **EP 1 641 153 B1**
(45) Veröffentlichungstag und Bekanntmachung des Hinweises auf die Patenterteilung: **12.09.2007**
(21) Anmeldenummer: 05020032.8
(22) Anmeldetag: 14.09.2005
(51) Int. Cl.: H04B 10/22, G02B 6/36, A61B 6/00

(54) **Optischer Drehübertrager mit Reinigungsvorrichtung**
Optical rotary transmitter with a cleaning device
Transmetteur rotatif optique avec un dispositif de nettoyage

(30) Priorität: 02.12.2004 DE 102004058293; 24.09.2004 DE 102004046775; 24.09.2004 DE 102004046777; 02.12.2004 DE 102004058292; 24.09.2004 DE 102004046774; 02.12.2004 DE 102004058291
(43) Veröffentlichungstag der Anmeldung: 29.03.2006
(60) Teilanmeldung: 06008583.4
(73) Patentinhaber: Schleifring und Apparatebau GmbH, 82256 Fürstenfeldbruck (DE)
(72) Erfinder: Schilling, Harry, 85072 Eichstätt (DE); Tartler, Thomas, 82272 Moorenweis (DE); Thiele, Hans, 80689 München (DE); Lohr, Georg, 82223 Eichenau (DE); Hutterer, Rainer, 81247 München (DE); Rank, Matthias, 93497 Wilmering (DE)
(74) Vertreter: Lohr, Georg

(56) Entgegenhaltungen:
- DE-A1- 3 704 887
- DE-A1- 10 256 634
- US-A- 4 900 117

## Beschreibung

### Technisches Gebiet

Die Erfindung betrifft eine Vorrichtung zur Übertragung optischer Signale zwischen gegeneinander drehbaren Einheiten. Derartige Vorrichtungen werden vorzugsweise in Computertomografen eingesetzt.

### Stand der Technik

Zur Übertragung optischer Signale zwischen gegeneinander drehbaren Einheiten, insbesondere mit einem freiem Innendurchmesser sind verschiedene Vorrichtungen bekannt. Grundsätzlich besteht hierin das Problem, ein Mittel zum Transport von Licht entlang des Umfangs der Vorrichtung sowie geeignete Mittel zur ein- und Auskopplung von Licht zu gestalten. Zum Einsatz in Computertomografen müssen derartige Vorrichtungen große freie Innendurchmesser in einer Größenordnung von 1 Meter aufweisen. Die Umfangsgeschwindigkeit bei der Rotation kann in einer Größenordnung von 20 m/s liegen. Gleichzeitig sollten Datenraten mit über 1 Gigabit pro Sekunde (GBaud) möglich sein.

Nahezu alle Arten von optischen Drehübertragern reagieren empfindlich auf verschmutzungen im Bereich des optischen Signalpfades. Auch in relativ sauberen Umgebungen, in denen beispielsweise Computertomografen eingesetzt werden, treten Staubpartikel auf. So wird beispielsweise in Computertomografen zur Energieversorgung der Röntgenröhre meist ein Schleifring mit einer Messingbahn und darauf laufenden Kohlebürsten eingesetzt. Diese Kohlebürsten werden im Betrieb durch das Schleifen auf der Messingbahn abgerieben und verursachen so einen extrem feinen Staub, welcher auch in feinste Öffnungen eindringen kann.

In der WO 03/069392 ist eine Vorrichtung zur breitbandigen Signalübertragung mittels eines in Längsrichtung geteilten Lichtleiters offenbart. Es ist der Inhalt der WO 03/069392 durch Bezugnahme mit in dieses Dokument aufgenommen. Der Lichtleiter liegt hier frei und damit ungeschützt vor Staub und anderen verschmutzungen weitere Vorrichtungen sind aus DE 3704887 vorbekannt.

### Darstellung der Erfindung

Der Erfindung liegt die Aufgabe zugrunde, eine Vorrichtung zur Übertragung optischer Signale zwischen zwei gegeneinander drehbaren Einheiten, vorzugsweise zum Einsatz in Computertomographen, derart zu gestalten, dass Staub und anderen Verschmutzungen, insbesondere von Schleifbahnen, die Übertragungsqualität nicht beeinträchtigen können.

Die erfindungsgemäße Lösung dieser Aufgabe ist im unabhängigen Patent anspruch 1 angegeben. Weiterbildungen der Erfindung sind Gegenstand der abhängigen Ansprüche.

Eine erfindungsgemäße Vorrichtung umfasst einen optischen Drehübertrager mit Lichtleiter 3, welcher entlang einer Kreisbahn an einer ersten Einheit 1 angeordnet ist. Der Einfachheit halber wird hier nur ein Lichtleiter beschrieben. Selbstverständlich können auch mehrere Lichtleiter in einer erfindungsgemäßen Vorrichtung vorgesehen werden. Zum Abgriff der Signale aus dem Lichtleiter 3 ist an einer zweiten Einheit 2, welche gegenüber der ersten Einheit 1 drehbar gelagert ist, ein Lichtkoppler 5 vorgesehen. Die erste Einheit 1 und die zweite Einheit 2 sind derart ausgebildet, dass sie gemeinsam wenigstens einen Lichtleiter 3 umschließen. In dem umschlossenen Raum mit dem Lichtleiter 3 ergibt sich ein Innenbereich 35. Außerhalb der gesamten Anordnung besteht der Außenbereich 36. Zwischen Innenbereich und Außenbereich verlaufen Spalte 37, 38, welche durch die drehbare Anordnung der beiden Einheiten gegeneinander bedingt sind. Weiterhin ist eine Vorrichtung zur Separation des Innenbereichs gegenüber dem Außenbereich vorgesehen. Diese Vorrichtung ist derart ausgelegt, dass ein Eindringen von Schmutz und /oder Staubpartikeln aus dem Außenbereich in den Innenbereich erschwert oder verhindert wird. Sie arbeitet bevorzugterweise auf hydrodynamische Art. Die Vorrichtung wird in ihrem Innenbereich 35 mit einem erhöhten Luftdruck gegenüber dem Außenbereich 36 beaufschlagt, so dass Luft durch Öffnungen in der Oberfläche, wie Spalte nach außen strömt und das eindringen von Partikeln verhindert.

In einer besonders vorteilhaften Ausgestaltung der Erfindung wird diese hydrodynamische Vorrichtung aus Luft versorgt, welche bereits zum Betrieb einer hydrostatischen oder hydrodynamischen Lagerung, insbesondere einer Luftlagerung verwendet wurde.

In einer weiteren vorteilhaften Ausgestaltung der Erfindung wird durch wenigstens eine Sperrluftdüse 51,52 zusätzliche Sperrluft 55 in die Spalte 37 bzw. 38 geblasen. Diese Sperrluft sorgt zumindest für einen von dem Innenbereich in den Außenbereich verlaufenden Luftstrom, welcher ein Eindringen von störenden Partikeln erschwert. Für eine optimale Wirkung sollte die Höhe 57 eines Spalts 37 in einem Bereich von 0,03 bis 0,1 mm sein. Besonders vorteilhaft ist eine Höhe in einer Größenordnung von 0,6 mm. Ein Druck der Sperrluft 55 am Austrittsort durch die Sperrluftdüse von 0,2 bis 0,5 bar hat sich besonders bewährt. Zum Austritt des nach innen gehenden Anteils der Sperrluft sowie der optionalen Zuluft 54 ist vorteilhafterweise ein Abluftventil 53 vorgesehen. Zu einer verbesserten Verteilung der Sperrluft entlang des Umfangs der Anordnung ist es vorteilhaft, zusätzliche ringförmige Nuten im Bereich wenigstens eines Spalts 37 bzw. 38 vorzusehen. Ebenso ist es vorteilhaft, die Sperrluft an mehreren Stellen, vorzugsweise entlang des Kreisumfangs einzuspeisen. Besonders vorteilhaft ist es, beide Spalte 37 bzw. 38 durch Sperrluft gegenüber dem Eindringen von Schmutz und Staub abzudichten.

Eine andere vorteilhafte Ausgestaltung der Erfindung sieht vor, dass wenigstens eine Labyrinthdichtung 58a, 58b im Bereich der Spalte 37 bzw. 38 vorgesehen ist. Zur Ausbildung der Labyrinthdichtung weisen die erste Einheit sowie die zweite Einheit im Bereich der Spalte Vertiefungen, insbesondere ringförmige Nuten, sowie darin eingreifende Erhöhungen, insbesondere Stege auf der Gegenseite auf. Wesentlich ist, dass die Erhöhungen sowie die Vertiefungen ineinander greifen. Dadurch wird die Weglänge zwischen dem Außenbereich und den Innenbereich vergrößert. Weiterhin erhöht sich der Strömungswiderstand. Durch eine scharfkantige Ausbildung entstehen zusätzliche Wirbel, welche zu einer Ablagerung von Verschmutzungen im Labyrinth führen, vor diese in den Innenraum eindringen. Durch die Erhöhung des Strömungswiderstandes genügt eine geringere Luftmenge zur Speisung des Innenbereichs mit Sperrluft.

Eine weitere Ausgestaltung der Erfindung sieht Bürsten 60 zwischen dem Innenbereich und dem Außenbereich im Bereich der Spalte vor. Hierdurch kann eine relativ gute Dichtwirkung insbesondere in Verbindung mit engen Spalten bzw. Labyrinthen erreicht werden. Diese Ausgestaltung erlaubt auch einen Schutz des Innenraumes im Stillstand und ohne Luftversorgung. In den Bürsten angesammelter Staub und Schmutz kann durch einen höheren Luftdruck nach außen ausgeblasen werden.

In einer anderen Ausgestaltung der Erfindung sind Filzdichtungen 61a, 61b zwischen dem Innenbereich und dem Außenbereich im Bereich der Spalte vorgesehen. Auch hierdurch ergibt sich eine besonders gute Dichtwirkung, welche auch im Stillstand und ohne Luftversorgung aus dem Innenbereich anhält. Durch einen erhöhten Luftdruck von der Innenseite kann dafür gesorgt werden, dass ein eventuell notwendiges Schmiermittel nicht in den Innenbereich eindringen kann.

Eine weitere Ausgestaltung der Erfindung sieht den Einsatz von Gleitringdichtungen vor. Wenigstens ein Gleitring 62 mit passendem Gegenring 63 ermöglicht eine gute Dichtung zwischen Innenbereich und Außenbereich. Vorteilhafterweise werden zwei Gleitringdichtungen miteinander kombiniert, so dass sie einen abgeschlossenen Zwischenraum zwischen dem Innenbereich und dem Außenbereich ausbilden. Dieser Zwischenraum kann dann vorteilhafterweise mit einem Sperrmedium oder mit einem Schmiermittel bzw. Kühlmedium für die Gleitringdichtungen gefüllt werden. Dieses kann durch Öffnungen 66 zugeführt und durch Öffnungen 67 abgeführt werden. Da durch die Gleitringdichtungen ein Luftaustritt aus dem Innenraum nicht mehr möglich ist, ist ein Abluftventil 53 zur Abführung der Zuluft 54 notwendig. Eine solche Ausgestaltung mit Gleitringdichtungen ist weitgehend wartungsfrei. Entstehender Abrieb kann durch das Schmiermittel aufgenommen und abgeführt werden. Der Innenbereich ist auch bei Stillstand und ohne Luftversorgung vor Verunreinigung geschützt.

Eine andere Ausgestaltung der Erfindung sieht den Einsatz von wenigstens einer Radialwellendichtung vor. Wenigstens eine solche Radialwellendichtung 64 ist in einem Spalt zwischen Innenbereich und Außenbereich vorgesehen. Vorteilhafterweise sind zwei solcher Dichtungen hintereinander vorgesehen, so dass diese einen abgeschlossenen Zwischenraum ausbilden, welcher wie bereits zuvor dargestellt, mit einem Sperrmedium, einem Schmiermittel oder Kühlmittel gefüllt bzw. versorgt werden kann. Auch hier kann ein eventuell entstehende Abrieb durch dieses Mittel abgeführt werden. Ebenfalls ist hier der Innenbereich bei Stillstand und ohne Luftversorgung vor Verunreinigung geschützt.

In einer weiteren vorteilhaften Ausgestaltung der Erfindung ist eine lichtdurchlässige Abdeckung 71 vorgesehen, welche mit der ersten Einheit 1 fest verbunden ist. Diese lichtdurchlässige Abdeckung dichtet zusammen mit der ersten Einheit den Lichtleiter 3 gegenüber dem Außenbereich ab. Weiterhin ist zur optischen Verkopplung mit dem Lichtleiter 3 wenigstens ein optisches Element zur freien Strahlführung durch die lichtdurchlässige Abdeckung vorgesehen. Ebenso wie der ungeschützte Lichtleiter kann auch die lichtdurchlässige Abdeckung verschmutzen. Der Vorteil der lichtdurchlässigen Abdeckung liegt aber darin, dass diese beispielsweise als plane Fläche und damit leicht reinigbar ausgebildet werden kann. Weiterhin ist es vorteilhaft, wenn der freie durch die lichtdurchlässige Abdeckung hindurchtretende Lichtstrahl gegenüber dem im Lichtleiter 3 geführten Strahl aufgeweitet wird. Damit ist auch noch bei einzelnen auf der Oberfläche liegenden Staub bzw. Schmutzpartikeln eine Übertragung möglich. Weiterhin kann die Optik zur Übertragung durch die lichtdurchlässige Abdeckung so ausgebildet sein, dass die lichtdurchlässige Abdeckung außerhalb des Fokus, vorzugsweise in einem parallelen Lichtstrahl liegt.

In einer weiteren Ausgestaltung der Erfindung ist ein Staubfilter 73,74 zwischen der ersten Einheit und der zweiten Einheit im Bereich wenigstens eines Spalts vorgesehen. Das Filter schließt den Spalt zumindest für Staub und Schmutzpartikel dicht ab, lässt aber Luft von der Innenseite entweichen. Auch diese Ausbildung ist im Stillstand und ohne Luftversorgung dicht in Bezug auf Staub und Schmutzpartikel. Ein vollgesetztes Filter lässt sich leicht durch erhöhten Luftdruck von der Innenseite aus reinigen. Das Filter besteht vorzugsweise aus einem Gewebe, wie einer Gaze oder auch einem Filterpapier. Ein optimales zusätzliches Filter 75 weist ein größeres Fassungsvolumen auf und kann auch gröbere Verunreinigungen ausfiltern.

In einer weiteren vorteilhaften Ausgestaltung der Erfindung ist noch ein zusätzliches Filter 75 als elektrostatisches Filter ausgebildet.

Eine andere Ausgestaltung der Erfindung sieht eine Dichtmembran 80 an einer der beiden Einheiten vor, welche vor einem Hohlraum 81 angebracht ist und durch Beaufschlagung des Hohlraumes 81 mit Vakuum bzw. Druckluft 82 bewegt werden kann. Durch Beaufschlagung mit Druckluft kann die Dichtmembran an eine diese gegenüberliegende Fläche, welche vorzugsweise der anderen Einheit zugeordnet ist angenähert beziehungsweise angedrückt werden. Durch Beaufschlagung mit Vakuum wird die Luft zumindest teilweise aus dem Hohlraum gesaugt und die Membran in diesen hineingezogen, sodass sich der Abstand zur gegenüberliegende Fläche vergrößert und damit die Dichtung geöffnet wird. Vorteilhafterweise wird die Membran so dimensioniert, dass sie im Ruhezustand leicht an der gegenüberliegenden Fläche anliegt, um eine gewisse Mindestdichtung in diesem Ruhezustand zu erreichen. Weiterhin wird Vorteilhafterweise an der gegenüberliegenden Fläche eine Dichtung angebracht, um eine definierte Auflagefläche zu Dichtmembran zu herzustellen.

In einer weiteren erfindungsgemäßen Anordnung ist wenigstens ein Gleitkörper 2a vorgesehen, welcher zur Aufnahme eines zweiten Lichtkopplers 5 dient. Weiterhin ist dieser Gleitkörper mit einer magnetischen Lagerung versehen. Die magnetische Lagerung dient zur präzisen Führung entlang des Lichtleiters 3. Sie kann sowohl statisch als auch dynamisch ausgebildet sein. So können wahlweise Permanentmagnete als auch Elektromagnete zur Erzeugung der Magnetfelder vorgesehen sein. Weiterhin ist es vorteilhaft, eine zusätzliche, vorzugsweise elektronisch gesteuerte Lageregelung vorzusehen.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt in allgemeiner Form schematisch eine erfindungsgemäße Vorrichtung.
Fig. 2 zeigt schematisch eine erfindungsgemäße Vorrichtung in der Draufsicht.
Fig. 3 zeigt eine Reinigungseinheit in Form einer Einrichtung zum Ausblasen von Staub und Schmutzpartikeln.
Fig. 4 zeigt eine Reinigungseinheit in Form einer Saugvorrichtung.
Fig. 5 zeigt eine Reinigungseinheit in Form einer Bürsteneinheit.
Fig. 6 zeigt eine erfindungsgemäße Vorrichtung mit einer Abdichtung durch Sperrluft.
Fig. 7 zeigt eine erfindungsgemäße Vorrichtung mit einer Labyrinthdichtung.
Fig. 8 zeigt eine erfindungsgemäße Vorrichtung mit einer Überdruckabdichtung.
Fig. 9 zeigt eine Vorrichtung mit einer Bürstenabdichtung.
Fig. 10 zeigt eine Vorrichtung mit einer Filzdichtung.
Fig. 11 zeigt eine Vorrichtung mit einer Gleitringdichtung.
Fig. 12 zeigt eine Vorrichtung mit Radialwellendichtungen.
Fig. 13 zeigt eine Vorrichtung mit einer statischen Dichtung.
Fig. 14 zeigt eine Vorrichtung mit einem mechanischen Filter.
Fig. 15 zeigt eine Vorrichtung mit einem elektrostatischen Filter.
Fig. 16 zeigt eine Anordnung mit einer Membrandichtung.
Fig. 17 zeigt eine Vorrichtung mit seitlich angeordnetem elektrostatischen Filter.
Fig. 18 zeigt eine Vorrichtung zur Abstoßung geladener Staub- und Schmutzpartikel.
Fig. 19 zeigt eine Vorrichtung mit einer Rolle zur Aufnahme geladener Staub- und Schmutzpartikel.
Fig. 20 zeigt eine Vorrichtung mit einer kombinierten Micro-/Nano-Struktur beschichteten Oberfläche.

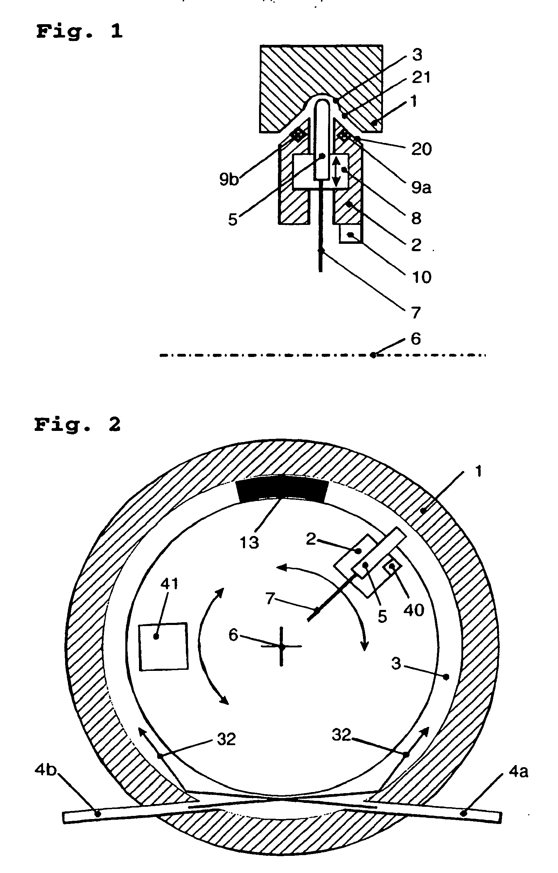

Fig. 1 zeigt in schematischer Form eine erfindungsgemäße Vorrichtung im Schnitt. Darin sind sowohl die erste Einheit 1 als auch die zweite Einheit 2 als Scheiben mit zentrischer Bohrung, welche um die Drehachse 6 drehbar gelagert sind, dargestellt. Der Lichtleiter 3 ist hier beispielhaft als auf der Innenseite verspiegelter Graben dargestellt. Er erstreckt sich um den gesamten Umfang der ersten Einheit. Im Eingriff mit diesem Graben ist ein zweiter Lichtkoppler 5, welcher an der zweiten Einheit 2 angeordnet ist. Dieser Lichtkoppler greift das in dem Lichtleiter geführte Licht ab und leitet es mit einer lichtleitenden Faser 7 weiter. Zur exakten Ausrichtung von Lichtleiter und zweitem Lichtkoppler in einer Achse ist eine Hydrodynamischen Lagerung sowie eine elektrodynamische Lageregelung vorgesehen. Die hydrodynamische Lagerung basiert auf einem dünnen Luftfilm, welche sich durch die Bewegung der beiden Einheiten gegeneinander zwischen der ersten Lagerfläche 21 und der zweiten Lagerfläche 20 ausgebildet wird. Zur Unterstützung sind beispielsweise zusätzliche Mittel zur Luftführung vorgesehen. Ein Aktuator 8 dient zur exakten Höheneinstellung des Lichtkopplers. Die Sensoren 9a und 9b dienen zur Ermittlung von Verschmutzungen.

Fig. 2 zeigt in schematischer Form eine erfindungsgemäße Vorrichtung in der Draufsicht. Eine erste Einheit 1 dient zur Aufnahme eines ringförmigen Lichtleiters 3. Dieser Lichtleiter ist beispielsweise ein auf der Innenseite verspiegelter Graben. Eine zweite Einheit 2 dreht sich gegenüber der ersten Einheit um die Drehachse 6. Die zweite Einheit enthält einen zweiten Lichtkoppler 5. Licht aus einem nicht dargestellten Sender wird bezogen auf das Modulationssignal gleichphasig mittels der beiden ersten Lichtkoppler 4a, 4b in den Lichtleiter 3 eingespeist. Das Licht vom ersten Lichtkoppler 4a läuft auf der rechten Seite der Abbildung bis zum Absorber 13. Gleichzeitig läuft das Licht des ersten Lichtkopplers 4b auf der linken Seite bis zum Absorber 13. Der Absorber ist symmetrisch in Bezug auf die Einkoppelstelle der ersten Lichtkoppler angeordnet, so dass die Lichtwege 32 auf beiden Seiten gleich lang sind. Der Abgriff des Lichts erfolgt mittels eines zweiten Lichtkopplers 5, welcher um die Drehachse 6 entlang der Bahn des Lichtleiters 3 drehbar gelagert ist und das abgegriffene Licht einem optischen Empfänger zuführt. Zur Vereinfachung ist der optische Empfänger ebenfalls nicht abgebildet. Eine Reinigungseinheit 40 ist in die zweite Einheit 2 integriert. Sie läuft mit dieser zweiten Einheit entlang dem Lichtleiter 3 und reinigt ihn entsprechend. Weiterhin ist eine unabhängige Reinigungseinheit 41 dargestellt, welche sich unabhängig von dem zweiten Lichtkoppler bewegen lässt.

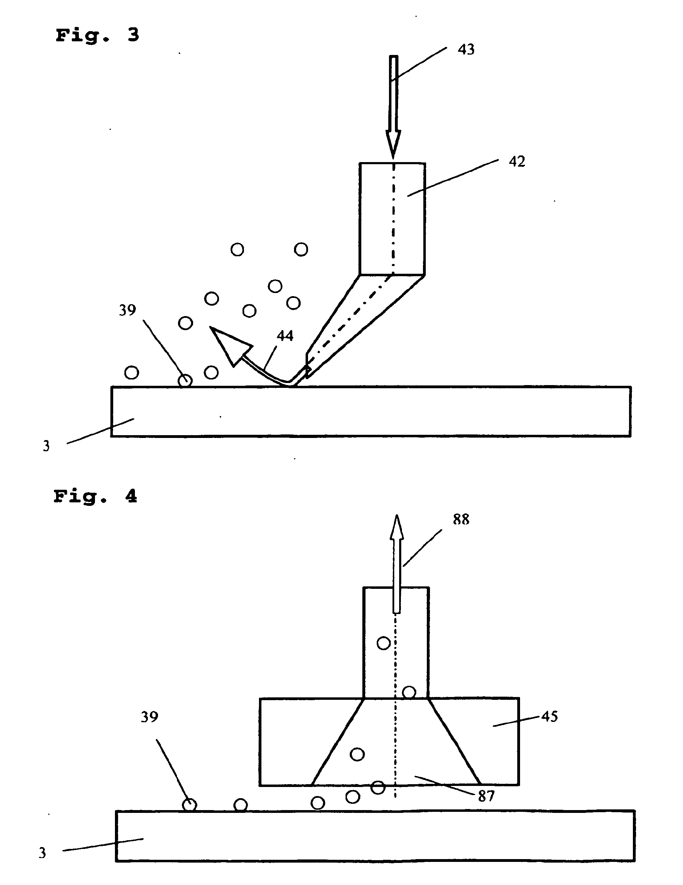

In Fig. 3 ist eine Reinigungseinheit in Form einer Einrichtung zum Ausblasen von Staub und Schmutzpartikeln dargestellt. Mittels einer Düse 42 wird ein Luftstrom 43 von einer Luftdruckquelle in Richtung des Lichtleiters Ziffer 3 geblasen. Der aus der Düse austretenden Luftstrom 44 wirbelt die Staubpartikel 39 auf und bläst diese vom Lichtleiter 3 fort.

Fig. 4 zeigt eine Reinigungseinheit in Form einer Saugvorrichtung. Ein Düsenblock 45, welcher in Form einer Düse ausgebildet ist, weist eine Absaugöffnung 87 auf, durch welche Luft von der Außenseite angesaugt wird. Der Luftstrom 88 reißt die Staubpartikel 39 mit und entfernt sie somit von der Oberfläche des Lichtleiters 3.

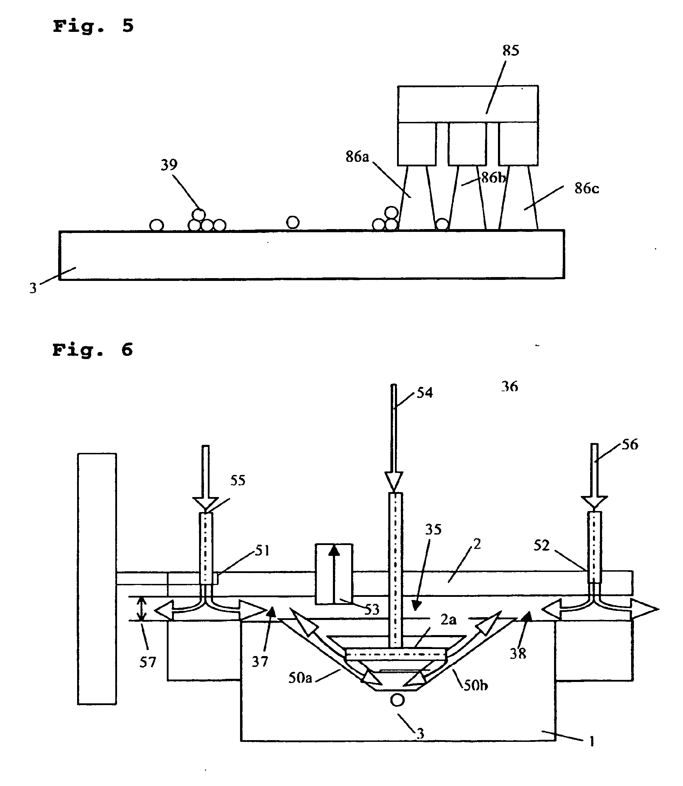

Fig. 5 zeigt eine Reinigungseinheit in Form einer Bürsteneinheit. Ein Bürstenträger 85 dient zur Aufnahme der Bürsten 86a, 86b, 86c. Die Bürsten werden entlang dem Lichtleiter 3 bewegt und kehren so auf der Oberfläche angelagerte Schmutzpartikel ab.

In Fig. 6 ist eine erfindungsgemäße Vorrichtung mit einer Abdichtung durch Sperrluft dargestellt. In dieser Darstellung ist zur Aufnahme des Lichtkopplers 5 ein Gleitkörper 2a, welcher mit der zweiten Einheit 2 verbunden ist vorgesehen. Dieser Gleitkörper 2a ist mit der zweiten Einheit 2 derart verbunden, dass er dieselbe Bewegung in Längsrichtung des Lichtleiters 3 ausführt, aber eine Stabilisierung in einer oder zwei Achsen senkrecht dazu bewirkt, so dass immer eine exakte Ausrichtung des Lichtkopplers 5 an der zweiten Einheit gegenüber dem Lichtleiter 3 gewährleistet ist. Eine besonders präzise und gleichzeitige Reibungsarme Lagerung dieses Gleitkörpers 2a gegenüber der ersten Einheit 1 lässt sich durch Luftlagerung erreichen. Eine solche Luftlagerung ist als besonders bevorzugtes Ausführungsbeispiel zur Erläuterung der erfindungsgemä-βen Ausgestaltungen dargestellt. Selbstverständlich ist auch jede andere Art der Lagerung, wie beispielsweise mit Gleitlagern oder Wälzlagern möglich. Zur Luftlagerung sind hier beispielhaft zwei Düsen 50a, 50b dargestellt, aus welchen ein Zuluftstrom 54 von einer nicht dargestellten Druckluftquelle in Richtung der ersten Einheit 1 abgegeben wird, um einen Luftfilm zwischen dem Gleitkörper 2a und der ersten Einheit 1 aufzubauen. Die durch die Düsen eingebrachte Luft strömt seitlich zwischen dem Gleitkörper 2a und der ersten Einheit 1 ab. Sie kann den in der Zeichnung dargestellten Hohlraum zwischen den Teilen der ersten Einheit 1 und der zweiten Einheit 2 durch das Abluftventil 53 verlassen.

Zur Abdichtung mittels Sperrluft ist nun wenigstens eine Sperrluftdüse 51 vorgesehen, welche einen Sperrluftstrom 55 in den Zwischenraum zwischen erste Einheit 1 und zweite Einheit 2 derart abgibt, dass dieser aus dem Spalt zwischen den beiden Einheiten nach außen hin austritt und somit ein Eindringen von Staub und anderen Schmutzpartikeln in den Spalt zwischen den beiden Einheiten verhindert. Zur Abdichtung der zweiten Seite ist noch eine zweite Sperrluftdüse 52 gespeist durch einen Sperrluftstrom 56 eingezeichnet. Um ein optimales Abdichtungsergebnis zu erhalten, kann der Spalt 57 zwischen der ersten Einheit 1 und der zweiten Einheit 2 optimiert werden. Besonders günstig ist ein Spalt in einer Größenordnung von 0,03 Millimetern bis 0,1 Millimeter, wobei sich ein Überdruck gegenüber der Umgebung von 0,2 bar bis 0,5 bar als vorteilhaft erwiesen hat. Gleiches gilt für den Spalt 58. Für den Gegenstand der Erfindung ist die exakte Anzahl der Spalte unwesentlich, da dies meist eine Frage der Definition ist. So könnten beispielsweise auch die beiden Spalte 57 und 58 als ein einziger Spalt zwischen den beiden Einheiten betrachtet werden. Erfindungswesentlich ist, dass wenigstens ein Spalt zwischen den beiden Einheiten besteht.

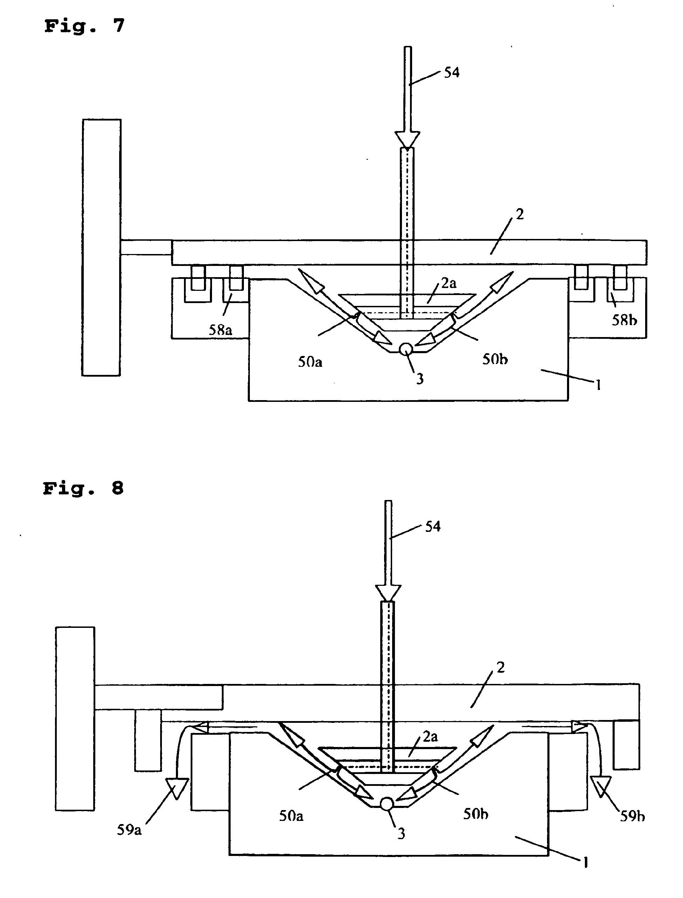

Fig. 7 zeigt eine erfindungsgemäße Vorrichtung mit einer Labyrinthdichtung. Hierbei wird das Eindringen von Staub und anderen Fremdkörpern in den Verschmutzung empfindlichen Bereich um den Lichtleiter 3 durch ein Labyrinth erschwert. Dieses Labyrinth wird vorzugsweise durch die Ausbildung einer ineinander greifenden Struktur aus Nuten beziehungsweise Stegen zwischen der ersten Einheit 1 und der zweiten Einheit 2 ausgebildet. In den hier dargestellten besonderen Ausführungsfall mit einer Luftlagerung tritt der in den Hohlraum eingeblasene Zuluftstrom 54 nach Austritt durch die Lagerung durch das Labyrinth nach außen und erschwert zusätzlich das Eindringen von Schmutz. Eine solche Labyrinthdichtung ist aber auch ohne den Luftstrom funktionsfähig.

Fig. 8 zeigt eine erfindungsgemäße Vorrichtung mit einer Überdruckabdichtung. Hierbei wird ähnlich, wie im vorhergehenden Beispiel, allerdings mit einer wesentlich größeren Luftmenge versucht, einen kontinuierlichen Luftstrom 59a beziehungsweise 59b nach außen hin aufrecht zu erhalten. Im Gegensatz zu Labyrinthdichtung ist die mechanische Ausführung hier wesentlich einfacher, allerdings ist ohne den Luftstrom nur eine sehr geringe Dichtwirkung zu erwarten. Dieses die vielen Anwendungsfällen aber nicht als besonders kritisch einzustufen, da im Ruhezustand einer Anlage auch der von außen aufgewirbelten Staub und damit das Verschmutzungsrisiko meist wesentlich geringer ist. Die Luftzufuhr kann wahlweise durch die Versorgung des Luftlagers oder durch zusätzliche Einströmöffnungen erfolgen. Vorzugsweise sind mehrere Einströmöffnungen auf dem Umfang der Anordnung verteilt vorgesehen.

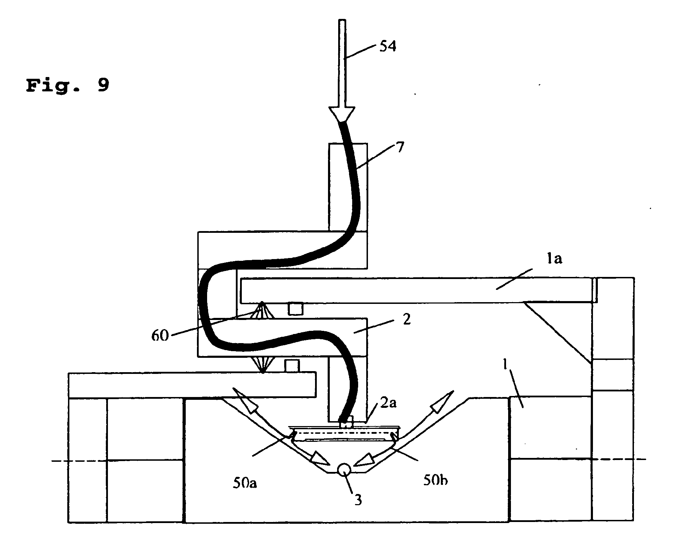

Fig. 9 zeigt eine Vorrichtung mit einer Bürstenabdichtung. Hierbei sind Bürsten 60 vorgesehen, welche ein Eindringen von Staub und Schmutz in den Zwischenraum zwischen der ersten Einheit 1 und der zweiten Einheit 2 verhindern. Zur Verbesserung der Dichtwirkung können vorteilhafterweise zusätzliche Barrieren oder Labyrinthe vorgesehen sein. In diesem Ausführungsbeispiel ist fest an der ersten Einheit 1 eine Abdeckung 1a angebracht, so dass nur ein relativ schmaler Bereich für die zweite Einheit 2 zur Durchführung einer lichtleitenden Faser 7 sowie der hier beispielhaft benötigten zu Luft 54 realisiert ist.

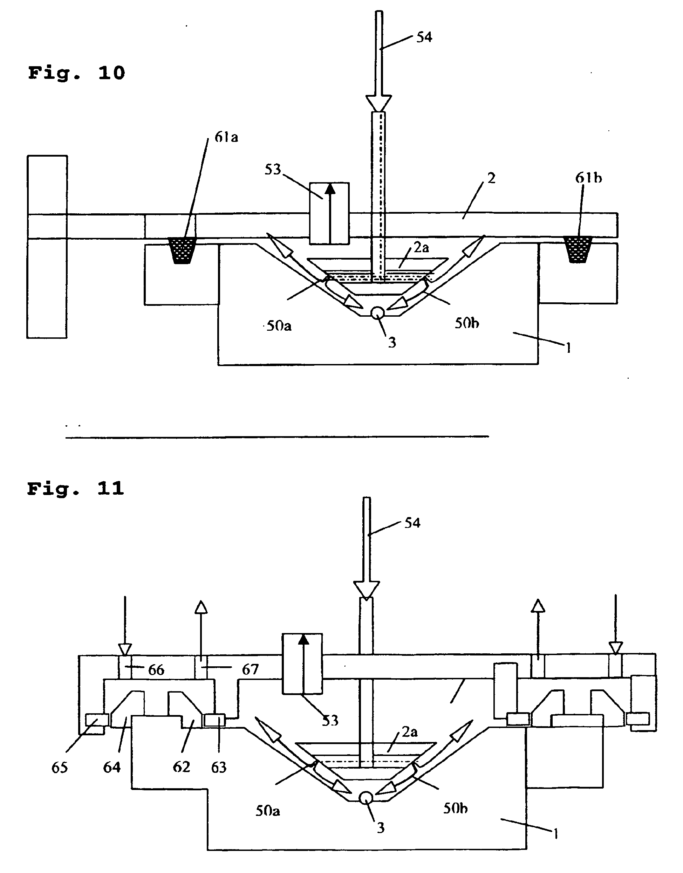

Fig. 10 zeigt eine Vorrichtung mit einer Filzdichtung. Eine solche Filzdichtung kann ähnlich wie die zuvor beschriebene Bürstendichtung ausgeführt werden. Hier ist wieder beispielhaft die planare Anordnung von der ersten Einheit 1 und der zweiten Einheit 2 dargestellt. Zur Unterstützung der Filzdichtung kann auch hier der interne Überdruck herangezogen werden.

Fig. 11 zeigt eine Vorrichtung mit einer Gleitringdichtung. Da die hier dargestellte Vorrichtung symmetrisch aufgebaut ist, wird nur auf die in der Abbildung links dargestellte Gleitringdichtung Bezug genommen. Grundsätzlich würde schon durch eine einfache Gleitringdichtung mit einem Gleitring 62 sowie dem Gegenring 63 eine Abdichtung des Innenraumes bewirken. Zur Verbesserung der Dichtwirkung ist noch ein zweiter Gleitring 64 mit dem entsprechenden Gegenring 65 vorgesehen. Weiterhin wird in den Zwischenraum, welcher durch die beiden Gleitringe und ihre Gegenringe abgeschlossen wird, durch einen Sperrlufteinlass 66 Sperrluft oder ein anderes Sperrmittel, wie beispielsweise eine Flüssigkeit eingebracht und durch den Sperrluftauslass 67 wieder abgeführt.

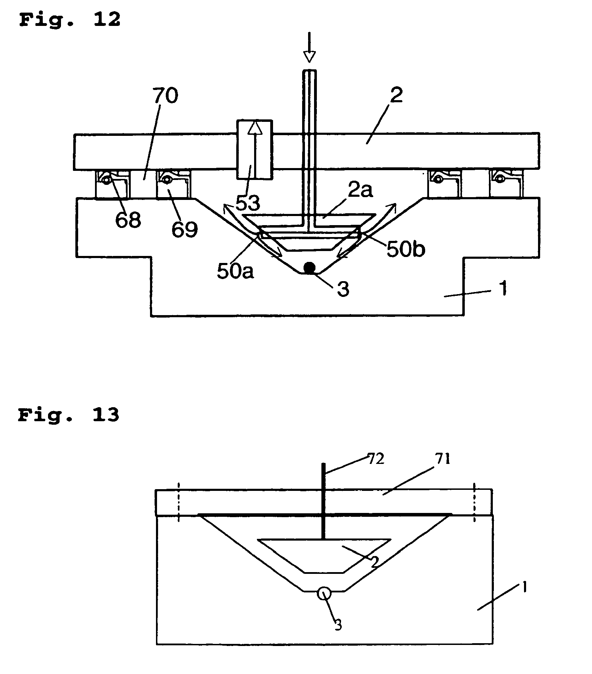

Fig. 12 zeigt eine Vorrichtung mit Radialwellendichtungen. Hierbei sind Zur Abdichtung des Innenraumes Radialwellendichtringe 68,69 vorgesehen. In den Zwischenraum 70 zwischen den Radialwellendichtringen kann beispielsweise ein Schmiermittel eingebracht werden. Ebenso kann dieser, wie vor der dargestellt, mit Sperrluft beaufschlagt werden.

Fig. 13 zeigt eine Vorrichtung mit einer statischen Dichtung. Hierbei wird im Gegensatz zu den zuvor beschriebenen Dichtsystemen der Raum über dem Lichtleiter 3 vollständig statisch abgedichtet. Hierzu wird eine lichtdurchlässige Abdeckung 71, wie beispielsweise eine Glasscheibe oder Plexiglasscheibe über dem Lichtleiter 3 angebracht. Das Licht wird vorzugsweise in freier Strahlführung 72 durch die lichtdurchlässige Abdeckung 71 gekoppelt. Ein der zweiten Einheit 2 zugeordneter Gleitkörper 2a, welcher die zur Verkopplung mit dem Lichtleiter 3 notwendigen optischen Elemente enthält, kann beispielsweise durch einen magnetischen Mitnehmer mechanisch mit der zweiten Einheit 2 verkoppelt werden.

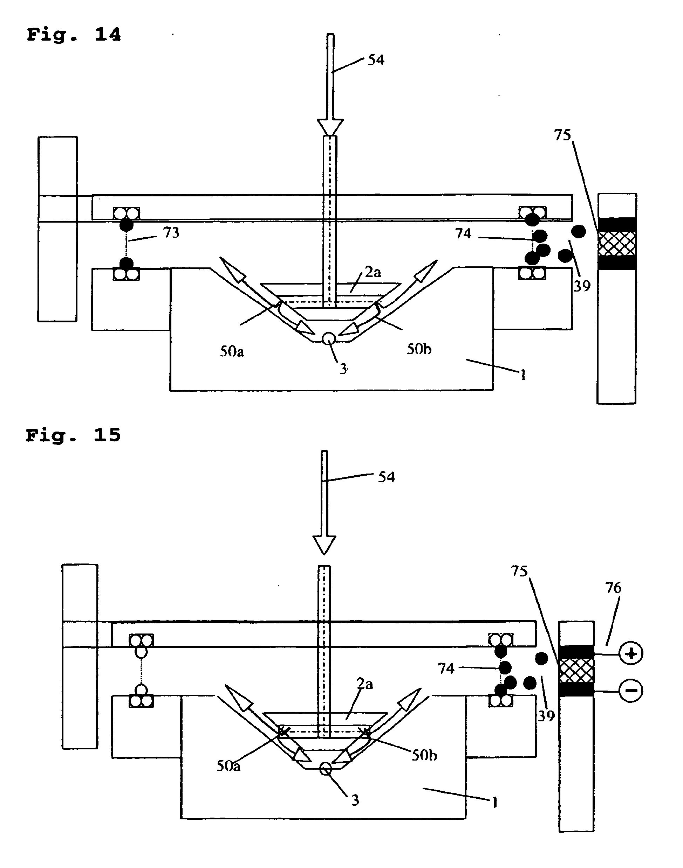

Fig. 14 zeigt eine Vorrichtung mit einem mechanischen Filter. Hier ist ein mechanisches Filter 73 bzw. 74 vorgesehen, um das Eindringen von Staub- bzw. Schmutzpartikeln in den Zwischenraum zwischen der ersten Einheit 1 und der zweiten Einheit 2 zu verhindern. Als Filtermaterial kann beispielsweise eine Gaze eingesetzt werden. Als Ergänzung kann noch ein weiteres Filter 75 mit einer größeren Oberfläche, beispielsweise ein Faltenbalg-Filter eingesetzt werden. Damit kann ein schnelles Zusetzen des Filters verhindert bzw. verzögert werden. Eine zusätzliche Unterstützung der Filterwirkung bzw. eine Reinigung des Filters kann durch den von innen kommenden Zuluftstrom 54 erfolgen.

Fig. 15 zeigt eine Vorrichtung mit einem elektrostatischen Filter. Ist grundsätzlich ähnlich wie die zuvor beschriebene Vorrichtung mit mechanischem Filter aufgebaut. Allerdings wird hier wahlweise an Stelle des mechanischen Filters kann 73,74 bzw. der Gaze oder aber auch an Stelle des zusätzlichen Filters 75 eine elektrostatisches Filter eingesetzt. Die Versorgung dieses elektrostatischen Filters erfolgt über die Hochspannungs-Anschlüsse 76.

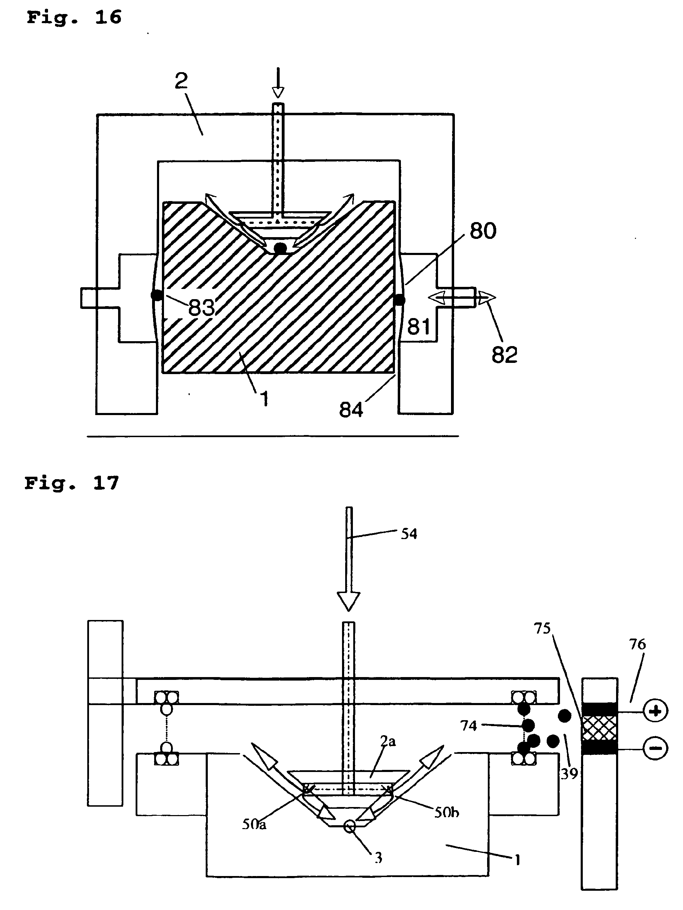

Fig. 16 zeigt eine Anordnung mit einer Membrandichtung. Hierbei ist eine Dichtmembran 80 vorgesehen, welche vor einem Hohlraum 81 angeordnet ist. Durch Vakuum bzw. Druckluft 82 kann nun die Lage der Dichtmembran verändert werden. Im gezeigten Bild kann diese durch Beaufschlagung mit Druckluft an die Dichtung 83 des ersten Teils angedrückt und durch Beaufschlagung mit Vakuum abgehoben werden. Das Spaltmaß 84 gibt den Hub der Dichtmembran an. In der dargestellten Ausführungsform ist im Ruhezustand die entlastete Membran so dimensioniert, dass sie gerade an dem Dichtring anliegt. Damit ist auch im Ruhezustand eine gewisse minimale Abdichtung sichergestellt. Zur Erhöhung der Dichtwirkung kann nun der Hohlraum bzw. die Rückseite der Membran mit Überdruck beaufschlagt werden. Im Betrieb wird durch Vakuum die Luft aus dem Hohlraum entzogen, so dass sich die Dichtmembran von der Dichtung abhebt und ein Betrieb bei minimaler Reibung möglich ist.

Fig. 17 zeigt eine Vorrichtung mit einem seitlich angebrachten elektrostatischen Filter. Durch den Zuluftstrom 54, welcher durch die Düsen 50a und 50b in dem Gleitkörper 2a der zweiten Einheit 2 ausströmt und den für die Luftlagerung notwendigen Luftfilm bildet, wird eine Strömung aus dem Lagerungs-Bereich nach außen erzeugt. Die durch diese Strömung mitgerissenen Staub und Schmutzpartikel 39 werden von einem elektrostatischen Filter 75 ausgefiltert. Der Filter 74 ist ein zusätzlicher Filter aus Gaze, um den Innenbereich der Lagerung gegen Einbringen von Staub von Schmutz zu schützen. Der elektrostatische Filter 75 wird über die Spannungsanschlüsse 76 gespeist. Zur Speisung kann ein Hochspannungsnetzteil oder eine Vorrichtung zur Erzeugung der hohen Spannung durch elektrostatisches Aufladung, z.B. auf Grund von Reibungselektrizität durch die Bewegung der ersten und zweiten Einheit gegeneinander vorgesehen werden.

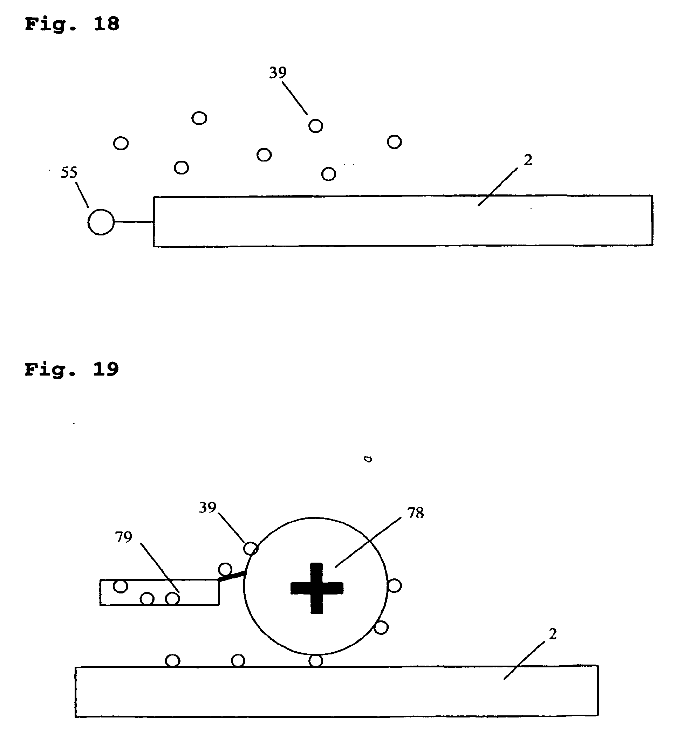

Fig. 18 zeigt eine andere Art der elektrostatischen Reinhaltung der Oberfläche insbesondere der zweiten Einheit 2. Hierbei wird diese durch eine statische Spannungsversorgung 90 mit Hochspannung beaufschlagt, so dass geladene Staub- und Schmutzpartikel 39 von dieser abgestoßen werden. Es kann eine zusätzliche Fläche mit umgekehrter Polarität vorgesehen werden, welche nun diese Partikel anzieht und auch festhält. Hierzu kann die Oberfläche eine Beschichtung, vorzugsweise aus einem Polymer mit einer hohen Adhäsion aufweisen.

Fig. 19 zeigt eine Vorrichtung mit einer Rolle zur Aufnahme geladener Staub- und Schmutzpartikel. Hierbei wird eine Rolle 78, welche beispielsweise durch eine Spannungsversorgung aufgeladen wird, in der Nähe der zu reinigenden Oberfläche, bevorzugt der zweiten Einheit bewegt. Diese nimmt nun geladene Staub- und Schmutzpartikel auf. Damit immer eine unverschmutzte Oberfläche der Rolle zur Verfügung steht, wird diese ebenfalls mit einer Drehbewegung weiterbewegt. Im vorliegenden Beispiel ist die Drehrichtung entgegen dem Uhrzeigersinn. Weiterhin ist ein Abscheider 79 vorgesehen, welcher die auf der Rolle befindlichen Staub- und Schmutzpartikel aufnimmt. Die Oberfläche der Rolle kann unterschiedlich gestaltet sein. So kann die Rolle eine durchgängig leitende Oberfläche aufweisen, wobei sie in ihrer Gesamtheit von einer Hochspannungsversorgung gespeist wird.-Ebenso kann sie aber auch ähnlich wie die bekannten Fotoleitertrommeln in Fotokopierern oder Laserdruckern ausgebildet sein, wobei die Aufladung der Oberfläche von einer in der Nähe der Oberfläche angebrachten Coronaelektrode aus erfolgt.

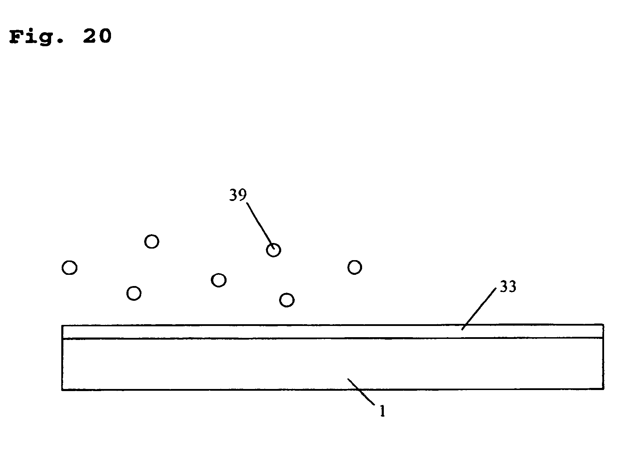

In Figur 20 ist noch eine kombinierte Micro-/NanoBeschichtung 33 dargestellt, welche durch einen Effekt, wie er durch die Lotus-Blüte bekannt ist, ein Ansetzen von Verunreinigungen 39 auf der Oberfläche verhindert. Eine solche Struktur kann wahlweise an der ersten Einheit 1, der zweiten Einheit 2 oder an anderen Teilen, wie dem Lichtleiter 3 angebracht sein. Hierzu ist vorzugsweise eine Mikrostruktur mit Erhebungen im Mikrometer-Bereich kombiniert mit einer darüber liegenden Nano-Struktur mit Erhebungen im Nanometer-Bereich. Eine derart beschichtete Oberfläche kann nun so ausgeführt werden, dass beispielsweise Verunreinigungen vollständig von den kritischen Bereichen der Oberfläche (optische Komponenten) weggeblasen werden können. Ebenso könnten diese gezielt in einen Auffangbehälter oder in ein Auffangfilter geleitet werden.

Selbstverständlich können die hier beschriebenen verschiedenen Ausgestaltungen der Erfindung miteinander kombiniert werden, um eine bessere Filterung von Schmutz- und Staubpartikeln zu erreichen.

### Bezugszeichenliste

- 1: Erste Einheit
- 1a: Abdeckung
- 2: Zweite Einheit
- 3: Lichtleiter
- 4: Erster Lichtkoppler an der ersten Einheit
- 5: Lichtkoppler an der zweiten Einheit
- 6: Drehachse der Drehung zwischen erster und zweiter Einheit
- 7: Lichtleitende Faser
- 8: Aktuator
- 9: Sensor
- 10: Steuereinheit
- 11: Referenzspur
- 13: Absorber
- 20: zweite Lagerfläche
- 21: erste Lagerfläche
- 32: Lichtstrahl
- 33: Mikro / Nanobeschichtung
- 35: Innenbereich
- 36: Außenbereich
- 37: erster Spalt
- 38: zweiter Spalt
- 39: Staubpartikel bzw. Schmutzschicht
- 40: in die zweite Einheit integrierte Reinigungseinheit
- 41: unabhängige Reinigungseinheit
- 42: Düse
- 43: Luftstrom von einer Luftdruckquelle
- 44: Luftstrom aus der Düse auftretend
- 45: Düsenblock
- 46: Staubpartikel bzw. Schmutzschicht
- 50: Düse
- 51: erste Sperrluftdüse
- 52: zweite Sperrluftdüse
- 53: Abluftventil
- 54: Zuluftstrom
- 55: erster Sperrluftstrom
- 56: zweiter Sperrluftstrom
- 57: Spalthöhe zwischen erster und zweiter Einheit
- 58: Labyrinth
- 59: Luftstrom
- 60: Bürsten
- 61: Filzring
- 62: Gleitring
- 63: Gegenring
- 64: Gleitring
- 65: Gegenring
- 66: Sperrlufteinlass
- 67: Sperrluftauslass
- 68: Radialwellendichtring
- 69: Radialwellendichtring
- 70: Kammer für Schmiermittel
- 71: Abdeckung - lichtdurchlässig
- 72: Lichtstrahl
- 73: Filter (Gaze)
- 74: Filter (Gaze)
- 75: Filter
- 76: Hochspannungs-Anschlüsse
- 78: Rolle
- 79: Abscheider
- 80: Dichtmembran
- 81: Hohlraum
- 82: Vakuum
- 83: Dichtung
- 84: Spaltmaß
- 85: Bürstenträger
- 86: Bürsten
- 87: Absaugöffnung
- 88: Luftstrom mit Schmutzpartikeln
- 90: Statische Spannungsversorgung

## Patentansprüche

1. Vorrichtung mit einem freien Innenraum zum Einsatz in Computertomographen zur Übertragung modulierter optischer Signale zwischen einer ersten Einheit (1) und einer zweiten Einheit (2), wobei die erste Einheit gegenüber der zweiten Einheit drehbar gelagert ist, umfassend
- einen Lichtleiter (3) entlang einer Kreisbahn an der ersten Einheit,
- wenigstens einen mit dem Lichtleiter verbundenen ersten Lichtkoppler (4) zur Lichtein- bzw. Auskopplung in den Lichtleiter,
- wenigstens einen zweiten Lichtkoppler (5), welcher an der zweiten Einheit angeordnet ist, und gegenüber dem Lichtleiter beweglich ist, zur Lichtein- bzw. Auskopplung in den Lichtleiter,
**dadurch gekennzeichnet, dass**
die erste Einheit und die zweite Einheit zusammen wenigstens einen Lichtleiter (3) umschließen, so dass sich durch die Umschließung ein Innenbereich (35) ergibt, welcher durch wenigstens zwei Spalte (37,38) zwischen den beiden Einheiten mit einem die gesamte Anordnung umgebenden Außenbereich (36) verbunden ist, und das weiterhin eine hydrodynamische Vorrichtung zur Separation des Innenbereichs (35) gegenüber dem Außenbereich (36) vorgesehen ist, die derart ausgelegt ist, dass ein Eindringen von Schmutz und /oder Staubpartikeln durch die Spalte aus dem Außenbereich in den Innenbereich erschwert oder verhindert wird, sowie durch Beaufschlagung des Innenbereichs (35) mit einem erhöhten Luftdruck gegenüber dem Außenbereich (36), so dass Luft ausgehend von dem Innenbereich in Richtung des Außenbereichs strömt und ein Eindringen von Schmutzpartikeln verhindert.

2. Vorrichtung nach Anspruch 1,
**dadurch gekennzeichnet, dass**
die hydrodynamische Vorrichtung zur Separation aus Luft (54) versorgt wird, welche bereits zum Betrieb einer hydrostatischen oder hydrodynamischen Lagerung des Gleitkörpers (2a) verwendet wurde.

3. Vorrichtung nach Anspruch 1,
**dadurch gekennzeichnet, dass**
wenigstens eine Sperrluftdüse (51) vorgesehen ist, welche von einem Sperrluftstrom (55) gespeist wird und Luft in den Bereich wenigstens eines Spalts (37) abgibt, wobei wenigstens einen Anteil der abgegebenen Luft in Richtung des Außenbereichs strömt.

4. Vorrichtung nach Anspruch 1,
**dadurch gekennzeichnet, dass**
sowohl die erste Einheit als auch die zweite Einheit zwischen dem Innenbereich (35) und dem Außenbereich (36) im Bereich wenigstens eines Spalts (37) gegenseitig ineinander greifende Erhöhungen bzw. Vertiefungen (58a, 58b) aufweisen.

5. Vorrichtung nach Anspruch 1,
**dadurch gekennzeichnet, dass**
zwischen dem Innenbereich (35) und dem Außenbereich (36) im Bereich wenigstens eines Spalts (37) wenigstens eine Bürste (60) zur Abdichtung vorgesehen ist.

6. Vorrichtung nach Anspruch 1,
**dadurch gekennzeichnet, dass**
zwischen den Innenbereich (35) und dem Außenbereich (36) im Bereich wenigstens eines Spalts (37) wenigstens eine Filzdichtung (61a, 61b) zur Abdichtung vorgesehen ist.

7. Vorrichtung nach Anspruch 1,
**dadurch gekennzeichnet, dass**
zwischen den Innenbereich (35) und dem Außenbereich (36) im Bereich wenigstens eines Spalts (37) wenigstens eine Gleitringdichtung (62,63) zur Abdichtung vorgesehen ist.

8. Vorrichtung nach Anspruch 1,
**dadurch gekennzeichnet, dass**
zwischen den Innenbereich (35) und dem Außenbereich (36) im Bereich wenigstens eines Spalts (37) wenigstens eine Radialwellendichtung (64) zur Abdichtung vorgesehen ist.

9. Vorrichtung nach Anspruch 1,
**dadurch gekennzeichnet, dass**
eine lichtdurchlässige, fest mit der ersten Einheit (1) verbundene, lichtdurchlässige Abdeckung (71) vorgesehen ist, welche den Lichtleiter (3) zusammen mit der ersten Einheit (1) dicht umschließt, und optischen Elemente zur Ausbildung eines freien Lichtstrahls (72) durch die lichtdurchlässige Abdeckung (71) vorgesehen sind.

10. Vorrichtung nach Anspruch 1,
**dadurch gekennzeichnet, dass**
wenigstens ein Staubfilter (73,74) in Bereich wenigstens eines Spalts zwischen der ersten Einheit und der zweiten Einheit angeordnet ist.

11. Vorrichtung nach Anspruch 1,
**dadurch gekennzeichnet, dass**
wenigstens ein Staubfilter (75) als elektrostatisches Filter ausgebildet ist.

12. Vorrichtung nach Anspruch 1,
**dadurch gekennzeichnet, dass**
eine Membrandichtung (80) vorgesehen ist, welche durch Druckluft bzw. Vakuum gesteuert werden kann.

13. Vorrichtung nach Anspruch 1,
**dadurch gekennzeichnet, dass**
wenigstens ein Gleitkörper 2a zur Aufnahme eines zweiten Lichtkopplers (5) vorgesehen ist, welcher eine magnetische Lagerung aufweist.

## Claims

1. Device having an unobstructed inner space, for use in a computer tomograph for transmitting modulated optical signals between a first unit (1) and a second unit (2), the first unit being supported to be rotatable relative to the second unit, comprising:
- a light guide (3) disposed along a circular track on the first unit;
- at least one first light coupler (4) connected with the light guide for coupling light into or out of the light guide;
- at least one second light coupler (5) disposed on the second unit to be movable relative to the light guide, for coupling light into or out of the light guide;
**characterized in that**
the first unit and the second unit jointly enclose at least one light guide (3) so that the enclosure results in an inner region (35) that communicates with an outer region surrounding the entire arrangement via at least two gaps between the two units, and that furthermore a hydrodynamic device for separating the inner region (35) from the outer region (36) is provided and configured so that an entry of dirt and/or dust particles from the outer region into the inner region through the gaps is rendered difficult or prevented, and also so that a subjection of the inner region (35) to an air pressure higher than that of the outer region (36) causes air to stream from the inner region in the direction of the outer region and prevent an entry of dirt particles.

2. Device according to claim 1,
**characterized in that**
the hydrodynamic device for separating the inner region from the outer region is supplied with air (54) that has already been used for operating a hydrostatic or hydrodynamic bearing of the sliding body (2a).

3. Device according to claim 1,
**characterized in that**
at least one seal-air nozzle (51) is provided which is fed by a seal-air stream (55) and emits air into the region of at least one gap (37), at least a portion of emitted air streaming in a direction towards the outer region.

4. Device according to claim 1,
**characterized in that**
both the first unit and the second unit have mutually intermeshing raised portions and recesses (58a, 58b) between the inner region (35) and the outer region (36), in the region of at least one gap (37).

5. Device according to claim 1,
**characterized in that**
at least one brush (60) is provided for sealing between the inner region (35) and the outer region (36) in the region of at least one gap (37).

6. Device according to claim 1,
**characterized in that**
at least one felt sealing means (61a, 61b) is provided for sealing between the inner region (35) and the outer region (36) in the region of at least one gap (37).

7. Device according to claim 1,
**characterized in that**
at least one sliding-ring sealing means (62, 63) is provided for sealing between the inner region (35) and the outer region (36) in the region of at least one gap (37).

8. Device according to claim 1,
**characterized in that**
at least one rotary-shaft sealing means (64) is provided for sealing between the inner region (35) and the outer region (36) in the region of at least one gap (37).

9. Device according to claim 1,
**characterized in that**
a light-transmitting cover (71) that is firmly attached to the first unit (1) is provided for enclosing in sealed manner the light guide (3) together with the first unit, and optical elements are provided for forming a free light beam (72) through the light-transmitting cover (71).

10. Device according to claim 1,
**characterized in that**
at least one dust filter (73, 74) is disposed between the first unit and the second unit in the region of at least one gap.

11. Device according to claim 1,
**characterized in that**
at least one dust filter (75) is designed to be an electrostatic filter.

12. Device according to claim 1,
**characterized in that**
a diaphragm sealing means (80) which can be controlled by pressurized air or vacuum is provided.

13. Device according to claim 1,
**characterized in that**
at least one sliding body (2a) is provided for accommodating a second light coupler (5), and comprises a magnetic bearing means.

## Revendications

1. Dispositif présentant un espace intérieur libre destiné à être utilisé dans des tomodensitomètres afin de transférer des signaux optiques modulés entre une première unité (1) et une seconde unité (2), la première unité étant logée en face de la seconde unité de manière à pouvoir pivoter, comprenant
- un conducteur optique (3) le long d'une trajectoire circulaire sur la première unité,
- au moins un premier coupleur optoélectronique (4) relié au conducteur optique destiné au couplage/découplage par lumière dans le conducteur optique,
- au moins un second coupleur optoélectronique (5) qui est disposé sur la seconde unité, et qui peut se déplacer vis-à-vis du conducteur optique, destiné au couplage/découplage par lumière dans le conducteur optique,
**caractérisé en ce que**
la première et la seconde unités entourent ensemble au moins un conducteur optique (3) de sorte qu'il résulte de par l'entourage une zone intérieure (35) qui est reliée par au moins deux fentes (37, 38) situées entre les deux unités à une zone extérieure (36) enveloppant l'ensemble de la disposition, et en outre, un dispositif hydrodynamique de séparation de la zone intérieure (35) est prévu vis-à-vis de la zone extérieure (36), lequel dispositif est conçu de telle manière qu'une pénétration d'impuretés et/ou de particules de poussière au travers des fentes de la zone extérieure dans la zone intérieure soit rendue difficile ou empêchée, ainsi que par l'alimentation de la zone intérieure (35) en une pression d'air élevée vis-à-vis de la zone extérieure (36) de sorte que l'air se répande à partir de la zone intérieure en direction de la zone extérieure et qu'une pénétration de particules d'impuretés soit empêchée.

2. Dispositif selon la revendication 1, **caractérisé en ce que** le dispositif hydrodynamique de séparation est alimenté en air (54), lequel a déjà été utilisé pour exploiter un logement hydrostatique ou hydrodynamique du corps coulissant (2a).

3. Dispositif selon la revendication 1, **caractérisé en ce qu'**au moins une buse d'air de blocage (51) est prévue, laquelle est alimentée par un courant d'air de blocage (55) et délivre de l'air dans la zone au moins d'une fente (37), au moins une partie de l'air délivré se répandant en direction de la zone extérieure.

4. Dispositif selon la revendication 1, **caractérisé en ce qu'**aussi bien la première unité que la seconde unité entre la zone intérieure (35) et la zone extérieure (36) présentent dans la zone au moins d'une fente (37) des élévations ou des cavités (58a, 58b) venant en prise l'une dans l'autre réciproquement.

5. Dispositif selon la revendication 1, **caractérisé en ce qu'**entre la zone intérieure (35) et la zone extérieure (36), au moins une brosse (60) est prévue dans la zone au moins d'une fente (37) à des fins d'étanchéité.

6. Dispositif selon la revendication 1, **caractérisé en ce qu'**entre la zone intérieure (35) et la zone extérieure (36), au moins un joint en feutre (61a, 61b) est prévu dans la zone au moins d'une fente (37) à des fins d'étanchéité.

7. Dispositif selon la revendication 1, **caractérisé en ce qu'**entre la zone intérieure (35) et la zone extérieure (36), au moins une garniture étanche à anneau glissant (62, 63) est prévue dans la zone au moins d'une fente (37) à des fins d'étanchéité.

8. Dispositif selon la revendication 1, **caractérisé en ce qu'**entre la zone intérieure (35) et la zone extérieure (36), au moins un joint pour arbre tournant (64) est prévu dans la zone au moins d'une fente (37) à des fins d'étanchéité.

9. Dispositif selon la revendication 1, **caractérisé en ce qu'**un couvercle (71) transparent, relié fixement à la première unité (1) est prévu, lequel couvercle enveloppe hermétiquement le conducteur optique (3) conjointement avec la première unité (1), et des éléments optiques sont prévus pour former un faisceau lumineux libre (72) au travers du couvercle (71) transparent.

10. Dispositif selon la revendication 1, **caractérisé en ce qu'**au moins un filtre à poussière (73, 74) est disposé dans la zone au moins d'une fente entre la première unité et la seconde unité.

11. Dispositif selon la revendication 1, **caractérisé en ce qu'**au moins un filtre antipoussières (75) est conçu comme filtre électrostatique.

12. Dispositif selon la revendication 1, **caractérisé en ce qu'**un joint de membrane (80) est prévu, lequel peut être commandé par de l'air comprimé ou du vide.

13. Dispositif selon la revendication 1, **caractérisé en ce qu'**au moins un corps coulissant (2a) est prévu pour recevoir un second coupleur optoélectronique (5), lequel présente un logement magnétique.
